# EUROPEAN PATENT APPLICATION

(11) **EP 4 831 278 A1**
(43) Date of publication of application: **28.10.2026**
(21) Application number: 24907560.7
(22) Date of filing: 20.12.2024
(51) Int. Cl.: C12N 5/071

(54) **CULTURE METHOD FOR PROMOTING ORGANOGENESIS OF KIDNEY WHILE RETAINING KIDNEY FORMATION REGION**

(30) Priority: 20.12.2023 JP 2023215087
(71) Applicant: Kyoto University, Kyoto-shi, Kyoto 606-8501 (JP); Rege Nephro Co., Ltd., Kyoto-shi, Kyoto, 606-8501 (JP)
(72) Inventor: OSAFUNE, Kenji, Kyoto-shi, Kyoto 606-8501 (JP); TSUJIMOTO, Hiraku, Kyoto-shi, Kyoto 606-8501 (JP)
(74) Representative: Hoffmann Eitle
(86) International application number: PCT/JP2024/045291
(87) International publication number: WO 2025/135177

(57) **Abstract**

The object is to produce a kidney tissue, and a kidney tissue is produced by co-culturing nephron progenitor cells, renal interstitial progenitor cells, and ureteric bud cells in a medium containing a Wnt signal activator.

## Description

### Technical Field

The present invention relates to a method for promoting kidney organogenesis. More specifically, the present invention relates to a kidney tissue-like culture having a kidney structure, a culture method therefor, and a method for producing the same.

### Background Art

The metanephros, which is the adult kidney of mammals, is formed from a plurality of embryonic renal progenitor cells, such as: nephron progenitor cells that give rise to nephron-constituting epithelial cells such as glomeruli and renal tubules; ureteric bud cells that give rise to collecting ducts and the lower urinary tract; and renal interstitial progenitor cells that give rise to renal interstitial cells, mesangial cells, vascular smooth muscle cells, and the like, but mainly through repeated self-renewal and differentiation of nephron progenitor cells, ureteric bud cells, and renal interstitial progenitor cells while maintaining a nephrogenic zone, organogenesis gradually occurs and develops/grows as an organ.

A culture method has been reported in which organogenesis is promoted by co-culturing nephron progenitor cells, ureteric bud cells, and renal interstitial progenitor cells derived from mouse fetal kidneys or mouse ES cells in a gel containing a medium including bovine serum (Non-Patent Document 1). Additionally, a culture method has been reported in which kidney organoids containing nephron progenitor cells, renal interstitial progenitor cells, and the like produced from human pluripotent stem cells are separated with a cell dissociation reagent and cultured in a gel containing a medium including FGF2, CHIR99021, retinoic acid, Y-27632, and GDNF, thereby deriving ureteric bud-like epithelium and promoting organogenesis (Non-Patent Document 2). However, to date, there has been no report of a culture method that promotes organogenesis while maintaining a nephrogenic zone when co-culturing human-derived nephron progenitor cells, ureteric bud cells, and renal interstitial progenitor cells, such as nephron progenitor cells, ureteric bud cells, and renal interstitial progenitor cells produced from human pluripotent stem cells such as human induced pluripotent stem (iPS) cells and human embryonic stem (ES) cells.

### Prior Art Documents

### Non-Patent Documents

[Non-Patent Document 1] Tanigawa S, Tanaka E, Miike K, Ohmori T, Inoue D, Cai C-L, et al., Nat Commun. 2022;13: 611.
[Non-Patent Document 2] Howden SE, Wilson SB, Groenewegen E, Starks L, Forbes TA, Tan KS, et al., Cell Stem Cell. 2021;28: 671-684.e6.

### Summary of the Invention

### Object to be Achieved by the Invention

An object of the present invention is to produce a kidney tissue having a more physiological structure by promoting organogenesis while maintaining a nephrogenic zone in the culture of nephron progenitor cells, renal interstitial progenitor cells, and ureteric bud cells. In particular, an object of the present invention is to produce a kidney tissue having a more physiological structure by promoting organogenesis while maintaining a nephrogenic zone in the culture of human-derived nephron progenitor cells, renal interstitial progenitor cells, and ureteric bud cells.

### Means for Achieving the Object

The present inventors conducted intensive studies to solve the above-mentioned problems. As a result, it was found that by co-culturing nephron progenitor cells, renal interstitial progenitor cells, and ureteric bud cells in the presence of a Wnt signal activator, organogenesis can be promoted while maintaining a nephrogenic zone, and a kidney tissue-like culture having a kidney structure can be produced.

That is, the present invention has the following features:
[1] A method for producing a kidney tissue-like culture, comprising a step of co-culturing nephron progenitor cells, renal interstitial progenitor cells, and ureteric bud cells in a medium containing a Wnt signal activator.
[2] The method according to [1], wherein the co-culture is performed by suspension culture.
[3] The method according to [2], wherein the suspension culture is performed in a state where an aggregate of nephron progenitor cells, renal interstitial progenitor cells, and ureteric bud cells is coated with an extracellular matrix.
[4] The method according to any one of [1] to [3], wherein the Wnt signal activator is a Wnt3a protein.
[5] The method according to any one of [1] to [4], wherein the medium further contains fibroblast growth factor 9 (FGF9).
[6] The method according to any one of [1] to [5], wherein the medium further contains retinoic acid (RA) and/or a derivative thereof, and glial cell line-derived neurotrophic factor (GDNF).
[7] The method according to [6], wherein the medium further contains alpha-albumin (Afamin) and R-Spondin 1 (RSPO1).
[8] The method according to any one of [1] to [7], wherein the kidney tissue-like culture has one or more of a mesangial structure, a branched ureteric bud structure, an S-shaped body-like structure, and a cap mesenchyme structure.
[9] The method according to any one of [1] to [8], wherein the nephron progenitor cells, the renal interstitial progenitor cells, and the ureteric bud cells are derived from a human.
[10] The method according to any one of [1] to [9], wherein one or more of the nephron progenitor cells, the renal interstitial progenitor cells, and the ureteric bud cells are cells induced from pluripotent stem cells.
[11] A kidney tissue-like culture, comprising human-derived cells and having a mesangial structure, a branched ureteric bud structure, an S-shaped body-like structure, and a cap mesenchyme structure.
[12] The kidney tissue-like culture according to [11], wherein: the mesangial structure is a structure containing PDGFRB- and/or GATA3-positive cells inside a glomerulus-like structure; the branched ureteric bud structure is a structure having two or more ureteric buds, wherein a tip portion of the ureteric bud contains RET-positive cells; the S-shaped body-like structure is a structure including an S-shaped body-like structure containing a proximal region and a distal region; and the cap mesenchyme structure is a structure that wraps the tip of the ureteric bud and contains SIX2- and/or PAX8-positive cells.

### Effects of the Invention

According to the method of the present invention, organogenesis can be promoted in nephron progenitor cells, renal interstitial progenitor cells, and ureteric bud cells while maintaining a nephrogenic zone, and a kidney tissue-like culture having a kidney structure can be produced. Further, according to the present invention, a kidney tissue-like culture having a kidney structure, particularly a human-derived culture, can be provided. Moreover, the culture of the present invention can particularly have a mesangial structure, a branched ureteric bud structure, and a cap mesenchyme structure. These structures are characteristic structures of the kidney during the organogenesis stage and are expected to further develop and grow as an organ.

### Brief Description of the Drawings

[FIG. 1] FIG. 1 is a diagram (photograph substituted for a drawing) showing the immunostaining results for SIX2, FOXD1, and CALB1 in a tissue-like culture on day 6 after kidney structure reconstruction was performed under CRFY conditions. The scale bar is 200 µm.
[FIG. 2] FIG. 2 is a diagram showing the analysis results of gene expression levels by quantitative RT-PCR (qT-PCR) for each progenitor cell marker gene in tissue-like cultures on day 6 after kidney structure reconstruction was performed under CRFY conditions, CRFY+G conditions, or RFY+G+WR conditions.
[FIG. 3] FIG. 3 is a diagram (photograph substituted for a drawing) showing the immunostaining results for SIX2, RET, and CALB1 in a tissue-like culture on day 6 after kidney structure reconstruction was performed under RFY+G+WR conditions. The scale bar is 200 µm.
[FIG. 4] FIG. 4 is a diagram (photograph substituted for a drawing) showing the immunostaining results for PAX8, SIX2, FOXD1, and CALB1 in a tissue-like culture on day 6 after kidney structure reconstruction was performed under RFY+G+WR conditions. The scale bar is 200 µm.
[FIG. 5] FIG. 5 is a diagram (photograph substituted for a drawing) showing the immunostaining results for BRN1, HNF4α, and CALB1 in a tissue-like culture on day 6 after kidney structure reconstruction was performed under RFY+G+WR conditions. The scale bar is 200 µm.
[FIG. 6] FIG. 6 is a diagram (photograph substituted for a drawing) showing the immunostaining results for PDGFRB, GATA3, MAFB, and NPHS1 in tissue-like cultures on day 6 and day 12 after kidney structure reconstruction was performed under RFY+G+WR conditions. The scale bar is 100 µm.
[FIG. 7] FIG. 7 is a diagram (photograph substituted for a drawing) showing the immunostaining result for PDFGRB in a cell mass obtained by aggregating induced nephron progenitor cells (NPC) under CRFY conditions and culturing them for 48 hours.
[FIG. 8] FIG. 8 is a diagram (photograph substituted for a drawing) showing the immunostaining results for the ureteric bud marker CK8 and the nephron progenitor cell marker SIX2 in a tissue-like culture on day 13 after kidney structure reconstruction was performed under RFY+G+WR conditions.

### Description of Embodiments

The present invention is described in detail below.

### <1> Method of the present invention

In one aspect, the present invention relates to a method for producing a kidney tissue-like culture, comprising a step of co-culturing nephron progenitor cells, renal interstitial progenitor cells, and ureteric bud cells in a medium containing a Wnt signal activator.

The "cell" of the present invention is, unless otherwise specified, preferably derived from a mammal, more preferably derived from a primate or a rodent, and even more preferably derived from a human or a mouse. Moreover, more preferably, the "cell" of the present invention is, unless otherwise specified, derived from a primate, and even more preferably derived from a human. Therefore, in the present invention, it is preferred that each of the nephron progenitor cells, the renal interstitial progenitor cells, and the ureteric bud cells is derived from a mammal, more preferably derived from a primate, and even more preferably derived from a human. That is, in the present invention, cells used for producing nephron progenitor cells, renal interstitial progenitor cells, and ureteric bud cells, such as induced pluripotent stem (iPS) cells and embryonic stem (ES) cells, and cells obtained from any of nephron progenitor cells, renal interstitial progenitor cells, and ureteric bud cells, such as mesangial cells and podocytes, are preferably derived from a mammal, more preferably derived from a primate, and even more preferably derived from a human.

Nephron progenitor cells (NPCs) are one type of progenitor cells for the formation of the metanephros, and give rise to nephron-constituting epithelial cells such as glomeruli and renal tubules. In addition to such properties, nephron progenitor cells may be characterized by the expression of marker genes. Specifically, nephron progenitor cells may be characterized by, for example, the expression of SIX2, which is a nephron progenitor cell marker gene, or the expression of SIX2 and OSR1, which is an intermediate mesoderm marker gene. The nephron progenitor cells are not particularly limited, and may be nephron progenitor cells isolated from a living body, or may be nephron progenitor cells induced from undifferentiated cells. Examples of nephron progenitor cells isolated from a living body include nephron progenitor cells isolated from an embryo. Examples of nephron progenitor cells induced from undifferentiated cells include nephron progenitor cells induced from pluripotent stem cells. The pluripotent stem cells are as described later. The method for inducing nephron progenitor cells from pluripotent stem cells is not particularly limited, and any known method may be used, or a method obtained by improving any known method may be used. Examples of such a method include the method described in Tsujimoto, H., et al. Cell Rep. 31, 107476, 2020, or a method obtained by improving this method. As a method obtained by improving the method described in Tsujimoto, H., et al. Cell Rep. 31, 107476, 2020, specifically, for example, re-seeding by a three-dimensional culture method on the 4th day of culture in the method and performing agitation culture can be mentioned.

Specifically, a method for inducing nephron progenitor cells from pluripotent stem cells may be carried out, for example, by first performing a step of inducing the posterior primitive streak (PPS) in pluripotent stem cells, then re-seeding the cells on about the 4th day after the start of PPS induction by a three-dimensional culture method, performing agitation culture for about 2 days in a medium containing a GSK-3β (glycogen synthase kinase 3β) inhibitor (such as CHIR99021), bFGF, and activin A, thereafter (about the 6th day after the start of PPS induction) replacing the medium with a medium containing retinoic acid (RA), NOGGIN, and FGF9 to perform culture, and further changing the medium to a medium containing FGF9 and a GSK-3β inhibitor (for example, CHIR99021) on about the 8th day after the start of PPS induction to perform culture for about 3 days. Further, when re-seeding by a three-dimensional culture method, a ROCK inhibitor (for example, Y-27632) may be added to the medium after re-seeding. More specifically, the step of inducing the posterior primitive streak (PPS) in pluripotent stem cells may be carried out, for example, by seeding pluripotent stem cells on a culture dish preferably coated with an extracellular matrix (specifically, for example, it may be a laminin fragment), performing culture for about 1 day (day 0) in a medium containing a GSK-3β inhibitor (for example, CHIR99021), RA, BMP4, and bFGF, switching to a medium containing CHIR99021, bFGF, and BMP7 to perform culture for about another 1 day (day 1), switching to a medium containing a GSK-3β inhibitor (for example, CHIR99021), BMP7, and a TGFβ inhibitor (for example, A83-01) to perform culture for about another 1 day (day 2), switching to a medium containing a GSK-3β inhibitor (for example, CHIR99021), bFGF, activin A, and a ROCK inhibitor (for example, Y-27632) to perform culture for about another 1 day (day 3), and collecting the cells on about the 4th day.

Renal interstitial progenitor cells (IPCs) are one type of progenitor cells for the formation of the metanephros, and are cells capable of differentiating into renal interstitial cells, renal erythropoietin-producing cells, mesangial cells, and renin-producing cells such as juxtaglomerular cells. In addition to such properties, renal interstitial progenitor cells may be characterized by the expression of marker genes. Specifically, renal interstitial progenitor cells may be characterized by, for example, the expression of FOXD1 (Forkhead Box D1), which is a renal interstitial progenitor cell marker gene, or the expression of FOXD1 as well as OSR1 and/or PDGFRB (platelet-derived growth factor receptor β). The renal interstitial progenitor cells are not particularly limited, and may be renal interstitial progenitor cells isolated from a living body, or may be renal interstitial progenitor cells induced from undifferentiated cells. Examples of renal interstitial progenitor cells isolated from a living body include renal interstitial progenitor cells isolated from an embryo. Examples of renal interstitial progenitor cells induced from undifferentiated cells include renal interstitial progenitor cells induced from pluripotent stem cells. The pluripotent stem cells are as described later. The method for inducing renal interstitial progenitor cells from pluripotent stem cells is not particularly limited, and any known method may be used, or a method obtained by improving any known method may be used. Examples of such a method include the method described in WO 2023/017848. The renal interstitial progenitor cells may be, for example, a by-product of inducing nephron progenitor cells. Examples of such a method include the methods described in JP2020031648A and WO2018216743A1.

Specifically, a method for inducing renal interstitial progenitor cells from pluripotent stem cells may be carried out, for example, by performing the step of inducing PPS in the above-mentioned pluripotent stem cells, next re-seeding the cells on about day 4 after the start of PPS induction so as to form aggregates (for example, aggregates may be formed by re-seeding in a U-bottom plate), culturing the cells for about 2 days in a medium containing a GSK-3β inhibitor (e.g., CHIR99021), bFGF, and activin A, thereafter (on day 6 after the start of PPS induction) exchanging the medium for a medium containing a GSK-3β inhibitor (e.g., CHIR99021), RA, a Smoothened (SMO) agonist (SAG), and IL-1β, but not containing bFGF, and culturing the cells, and on about day 8 exchanging the medium for a medium containing a GSK-3β inhibitor (e.g., CHIR99021), RA, SAG, and a TGFβ inhibitor (e.g., SB431542), but not containing bFGF, and culturing the cells for about 3 days.

Ureteric bud (UB) cells are one of the progenitor cells for the formation of the metanephros, and give rise to the collecting ducts and the lower urinary tract. Ureteric bud cells are also called nephric duct (ND) cells depending on their location in the tissue or the stage of development, but these cells do not have to be distinguished in vitro. That is, ureteric bud cells in the present invention may be paraphrased as ND cells. In addition to such properties, ureteric bud cells may be characterized by the expression of a marker gene. Specifically, ureteric bud cells may be characterized, for example, by the expression of RET, which is a ureteric bud cell marker gene. Ureteric bud cells are not particularly limited, and may be ureteric bud cells isolated from a living body or ureteric bud cells induced from undifferentiated cells. Examples of ureteric bud cells isolated from a living body include ureteric bud cells isolated from an embryo. Examples of ureteric bud cells induced from undifferentiated cells include ureteric bud cells induced from pluripotent stem cells. Pluripotent stem cells will be described later. A method for inducing ureteric bud cells from pluripotent stem cells is not particularly limited, and any known method may be used, or a method in which any known method is improved may be used. Examples of such a method include the methods described in Tsujimoto, H., et al. Cell Rep. 31, 107476, 2020 and Mae, S.I., et al. Cell Rep. 32. 2020. An example of a method for inducing ureteric bud cells from pluripotent stem cells includes a method of inducing them via anterior intermediate mesoderm. Specifically, a method for inducing anterior intermediate mesoderm from pluripotent stem cells may be carried out, for example, by seeding pluripotent stem cells on a culture dish coated with an extracellular matrix (specifically, which may be, for example, a laminin fragment), culturing the cells for about 1 day (day 0) in a serum-free medium in which activin A, a GSK-3β inhibitor (e.g., CHIR99021), BMP4, and bFGF are added to a basal medium such as Essential 6 (E6), switching to a medium containing a GSK-3β inhibitor (e.g., CHIR99021), bFGF, and BMP7 and further culturing for about 1 day (day 1), switching to a medium containing an ALK2/3 inhibitor (e.g., LDN193189), a TGFβ inhibitor (e.g., A83-01), retinoic acid or a derivative thereof (e.g., TTNPB), and FGF8 and further culturing for about 2 days (days 2 to 3), and switching to a medium containing a ROCK inhibitor (e.g., Y-27632) in addition to an ALK2/3 inhibitor (e.g., LDN193189), a TGFβ inhibitor (e.g., A83-01), retinoic acid or a derivative thereof (e.g., TTNPB), and FGF8 and further culturing for about 1 day (day 4). Specifically, a method for inducing ureteric bud cells from anterior intermediate mesoderm may be carried out, for example, by culturing anterior intermediate mesoderm for about 2 days in a medium such as E6 containing a GSK-3β inhibitor (e.g., CHIR99021), an ALK2/3 inhibitor (e.g., LDN193189), FGF8, GDNF, and retinoic acid or a derivative thereof (e.g., TTNPB), separating the cultured cells into single cells using a cell detachment agent (e.g., Accutase or the like), re-seeding the cells on a low-attachment plate, and further culturing the cells for about 2 days in an E6 medium containing a ROCK inhibitor (e.g., Y-27632) in addition to a GSK-3β inhibitor (e.g., CHIR99021), an ALK2/3 inhibitor (e.g., LDN193189), FGF8, GDNF, and retinoic acid or a derivative thereof (e.g., TTNPB) for induction. Further, the cells at this stage may be regarded as cells corresponding to ND cells. In addition, the cells after induction may be further cultured for about 6 days in a medium such as E6 containing a GSK-3β inhibitor (e.g., CHIR99021), an ALK2/3 inhibitor (e.g., LDN193189), FGF8, GDNF, retinoic acid or a derivative thereof (e.g., TTNPB), FGF1, EGF, and Matrigel.

In the present invention, nephron progenitor cells, renal interstitial progenitor cells, and ureteric bud cells may or may not be induced from pluripotent stem cells as described above. That is, regarding nephron progenitor cells, renal interstitial progenitor cells, and ureteric bud cells, one or more of these may be induced from pluripotent stem cells, or all of them may be induced from pluripotent stem cells.

Pluripotent stem cells are stem cells that have pluripotency capable of differentiating into many types of cells existing in a living body and also have proliferation ability, and encompass any cells that are induced to differentiate into a blastocyst-like structure. The pluripotent stem cells include, but are not particularly limited to, for example, embryonic stem (ES) cells, induced pluripotent stem (iPS) cells, embryonic germ cells (EG cells), and the like. Preferred pluripotent stem cells are iPS cells and ES cells. Further, the pluripotent stem cells may be naive pluripotent stem cells or primed pluripotent stem cells, and are not particularly limited. The origin of the pluripotent stem cells is preferably derived from a mammal, more preferably derived from a primate, and even more preferably derived from a human.

As ES cells, ES cells established by known methods can be used. Methods for establishing and maintaining ES cells are described, for example, in USP 5,843,780; Thomson JA, et al. (1995), Proc Natl. Acad. Sci. U S A. 92:7844-7848; Thomson JA, et al. (1998), Science. 282:1145-1147; H. Suemori et al. (2006), Biochem. Biophys. Res. Commun., 345:926-932; M. Ueno et al.

(2006), Proc. Natl. Acad. Sci. USA, 103:9554-9559; H. Suemori et al. (2001), Dev. Dyn., 222:273-279; H. Kawasaki et al. (2002), Proc. Natl. Acad. Sci. USA, 99:1580-1585; Klimanskaya I, et al. (2006), Nature. 444:481-485, and the like. As for human ES cell lines, for example, WA01 (H1) and WA09 (H9) are available from the WiCell Research Institute, and KhES-1, KhES-2, and KhES-3 are available from the Institute for Frontier Medical Sciences, Kyoto University (Kyoto, Japan).

Embryonic germ cells are cells established from primordial germ cells in the embryonic stage and have pluripotency similar to ES cells, and can be established by culturing primordial germ cells in the presence of substances such as LIF, bFGF, and stem cell factor (Y. Matsui et al. (1992), Cell, 70:841-847; J.L. Resnick et al. (1992), Nature, 359:550-551).

Induced pluripotent stem (iPS) cells are somatic cell-derived artificial stem cells that have characteristics substantially equivalent to those of ES cells, such as differentiation pluripotency and proliferation ability by self-renewal, and can be produced by introducing specific reprogramming factors into somatic cells in the form of DNA or proteins (K. Takahashi and S. Yamanaka (2006) Cell, 126:663-676; K. Takahashi et al. (2007), Cell, 131:861-872; J. Yu et al. (2007), Science, 318:1917-1920; Nakagawa, M. et al. (2008), Nat. Biotechnol. 26:101-106; International Publication WO 2007/069666). The reprogramming factors may be composed of a gene specifically expressed in ES cells, a gene product thereof, or non-coding RNA, or a gene playing an important role in maintaining the undifferentiated state of ES cells, a gene product thereof, or non-coding RNA, or a small molecule compound having an equivalent function. Numerous genes have been reported as genes included in the reprogramming factors and are not particularly limited, but examples thereof include Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tcl1, beta-catenin, Lin28b, Sall1, Sall4, Esrrb, Nr5a2, Tbx3, or Glis1, etc., and these may be used alone or in combination. The above reprogramming factors also include factors used for the purpose of increasing the establishment efficiency of iPS cells, and these can also be used as reprogramming factors: histone deacetylase (HDAC) inhibitors (e.g., small molecule inhibitors such as valproic acid (VPA), trichostatin A, sodium butyrate, MC 1293, M344, etc.; nucleic acid expression inhibitors such as siRNA and shRNA against HDAC, etc.), MEK inhibitors (e.g., PD184352, PD98059, U0126, SL327, and PD0325901), Glycogen synthase kinase-3 inhibitors (e.g., Bio and CHIR99021), DNA methyltransferase inhibitors (e.g., 5-azacytidine), histone methyltransferase inhibitors (e.g., small molecule inhibitors such as BIX-01294, etc.; nucleic acid expression inhibitors such as siRNA and shRNA against Suv39hl, Suv39h2, SetDBl, and G9a, etc.), L-channel calcium agonist (e.g., Bayk8644), butyric acid, TGFβ inhibitors or ALK5 inhibitors (e.g., LY364947, SB431542, 616453, and A-83-01), p53 inhibitors (e.g., siRNA and shRNA against p53), ARID3A inhibitors (e.g., siRNA and shRNA against ARID3A), miRNAs such as miR-291-3p, miR-294, miR-295, and mir-302, Wnt Signaling (e.g., soluble Wnt3a), neuropeptide Y, prostaglandins (e.g., prostaglandin E2 and prostaglandin J2), hTERT, SV40LT, UTF1, IRX6, GLIS1, PITX2, DMRTB1, and the like. iPS cells may be produced and used by introducing reprogramming factors into somatic cells as described above, or may be obtained from a cell bank or the like for use.

The method of the present invention includes a step of co-culturing nephron progenitor cells, renal interstitial progenitor cells, and ureteric bud cells. Co-culturing nephron progenitor cells, renal interstitial progenitor cells, and ureteric bud cells is not particularly limited as long as these cells are cultured in the same medium, but may be carried out, for example, by mixing and culturing nephron progenitor cells, renal interstitial progenitor cells, and ureteric bud cells. The mixing of nephron progenitor cells, renal interstitial progenitor cells, and ureteric bud cells is not particularly limited as long as a kidney tissue-like culture is obtained, but these cells may be dissociated into single cells respectively before being mixed, or cell masses with a certain number of cells may be mixed, or a combination thereof may be used. Preferably, nephron progenitor cells, renal interstitial progenitor cells, and ureteric bud cells are respectively dissociated into single cells before being mixed. The proportion of nephron progenitor cells, renal interstitial progenitor cells, and ureteric bud cells is not particularly limited as long as a kidney tissue-like culture is obtained, but the co-culture may be started at a ratio of 25-40 : 25-40 : 25-40 in terms of cell number, preferably at a ratio of 30-35 : 30-35 : 30-35, and even more preferably at a ratio of 1:1:1.

Co-culture of nephron progenitor cells, renal interstitial progenitor cells, and ureteric bud cells may preferably be carried out by suspension culture. Here, suspension culture means that cells are cultured in a state non-adherent to a culture substrate, and examples thereof include an embodiment of culturing using a low cell-attachment culture vessel.

In the suspension culture, aggregates of nephron progenitor cells, renal interstitial progenitor cells, and ureteric bud cells may be formed, and the aggregates may be subjected to a coating treatment. As the coating agent, an extracellular matrix is preferred, and examples thereof include substances such as collagen, proteoglycan, fibronectin, hyaluronic acid, tenascin, entactin, elastin, fibrin, and laminin, or fragments thereof. As a method for coating the aggregates of cells, for example, after starting suspension culture (e.g., after 48 hours), the aggregated cell masses may be moved to droplets formed of a medium containing 50 vol% of a coating agent, and after performing the coating treatment, the coating treatment may be stopped by additionally adding medium. At this time, the duration of the coating treatment is not particularly limited, but may be, for example, 30 minutes or more.

A medium for co-culturing nephron progenitor cells, renal interstitial progenitor cells, and ureteric bud cells can be prepared by adding a Wnt signal activator to a basal medium used for culturing animal cells, and may be prepared by further adding one or more of GDNF, FGF9, and retinoic acid (RA) and/or a derivative thereof as necessary. Moreover, it may be prepared by further adding alpha-albumin (Afamin) and/or R-spondin 1 (RSPO1) as necessary. Commercially available products can be used for these. It is preferred that the medium for co-culturing nephron progenitor cells, renal interstitial progenitor cells, and ureteric bud cells is prepared by adding a Wnt signal activator to a basal medium used for culturing animal cells, and further adding at least FGF9. Examples of the basal medium include IMDM medium, Medium 199 medium, Eagle's Minimum Essential Medium (EMEM) medium, αMEM medium, Dulbecco's Modified Eagle's Medium (DMEM) medium, Ham's F12 (F12) medium, RPMI 1640 medium, Fischer's medium, and mixed media thereof. The medium may contain serum (e.g., fetal bovine serum (FBS)) or may be serum-free. As necessary, it may contain one or more serum replacements such as, for example, albumin, transferrin, KnockOut Serum Replacement (KSR) (a serum replacement for ES cell culture) (Invitrogen), N2 supplement (Invitrogen), B27 supplement (Invitrogen), fatty acids, insulin, collagen precursors, trace elements, 2-mercaptoethanol, and 3'-thiolglycerol; and may also contain one or more substances such as lipids, amino acids, L-glutamine, GlutaMAX (Invitrogen), non-essential amino acids (NEAA), vitamins, growth factors, antibiotics, antioxidants, pyruvic acid, buffers, inorganic salts, and equivalents thereof. A medium optimized in advance for stem cell culture, such as ReproFF2 (ReproCELL) or StemFit AK02N medium (Ajinomoto Healthy Supply), may be used.

In the present invention, a medium for co-culturing nephron progenitor cells, renal interstitial progenitor cells, and ureteric bud cells (hereinafter also referred to as "the medium of the present invention") contains a Wnt signal activator. The Wnt signal activator is not particularly limited as long as it is a substance that acts on a receptor of the Wnt signaling pathway and activates Wnt signaling, and may include proteins, peptides, small molecule compounds, organic compounds, antibodies, and the like. Note that the "receptor of the Wnt signaling pathway" may include a co-receptor of the Wnt signaling pathway, such as LRP. The Wnt signal activator may preferably be a protein or a peptide. Specific examples of the Wnt signal activator include proteins encoded by the Wnt gene family (Wnt family proteins), such as Wnt1, Wnt2, Wnt3, Wnt3a, Wnt4, Wnt5a, Wnt6, Wnt7a, Wnt8a, Wnt9a, Wnt10a, Wnt11, and Wnt16; as the Wnt family proteins, Wnt3 or Wnt3a protein is preferred. Further, examples of the Wnt signal activator include Wnt agonists such as analogous peptides of Wnt family proteins, and specifically, for example, Foxy5 and the like. In a preferred embodiment, the Wnt signal activator may be Wnt3a protein or an analogous peptide of Wnt3a protein, and more preferably is Wnt3a protein. Wnt3a protein is available, for example, from MBL Co., Ltd., or may be prepared by oneself. The method for producing Wnt3a protein is not particularly limited and may be any known method, and examples thereof include a medium supernatant in which Wnt3a protein is expressed in a serum-free medium. In the case where Wnt3a is used as the Wnt signal activator, the concentration of Wnt3a in the medium of the present invention is not particularly limited as long as a kidney tissue-like culture can be produced, but may be, for example, 1 ng/mL or more, 5 ng/mL or more, 10 ng/mL or more, 50 ng/mL or more, 100 ng/mL or more, 200 ng/mL or more, 500 ng/mL or more, or 1,000 ng/mL or more; and may be 10,000 ng/mL or less, 1,000 ng/mL or less, 500 ng/mL or less, 200 ng/mL or less, 100 ng/mL or less, 50 ng/mL or less, or 10 ng/mL or less; or may be any non-contradictory combination thereof. Specifically, the concentration of Wnt3a in the medium of the present invention may be, for example, 1 ng/mL to 10,000 ng/mL, 10 ng/mL to 1,000 ng/mL, 50 ng/mL to 500 ng/mL, 100 ng/mL to 1,000 ng/mL, or 100 ng/mL to 500 ng/mL.

In the present invention, the medium for co-culturing nephron progenitor cells, renal interstitial progenitor cells, and ureteric bud cells may contain alpha-albumin (Afamin). It is known that Wnt family proteins such as Wnt3a are prevented from forming aggregates and are stabilized in the presence of alpha-albumin (Afamin). Therefore, from the above viewpoint, when the medium of the present invention contains a Wnt family protein such as Wnt3a, it preferably also contains alpha-albumin (Afamin). Afamin may be contained, for example, in a medium supernatant in which the above-mentioned Wnt3a protein is expressed in a serum-free medium. A medium supernatant containing Afamin and Wnt3a, i.e., Afamin/Wnt3a Conditioned Medium, is available, for example, from MBL Co., Ltd. In the case where Wnt3a protein is used, the concentration of Wnt3a protein in the medium of the present invention is not particularly limited as long as a kidney tissue-like culture is obtained, but may be, for example, in terms of Afamin/Wnt3a Conditioned Medium, 1 vol% or more, 3 vol% or more, 5 vol% or more, 8 vol% or more, or 10 vol% or more; and may be 20 vol% or less, 18 vol% or less, 15 vol% or less, 12 vol% or less, or 10 vol% or less; or may be any non-contradictory combination thereof. Specifically, the concentration of Wnt3a protein in the medium of the present invention may be 1 to 20 vol%, 3 to 18 vol%, 5 to 15 vol%, 8 to 12 vol%, or 10 vol% in terms of Afamin/Wnt3a Conditioned Medium. The concentration of Afamin in the medium of the present invention is also not particularly limited as long as a kidney tissue-like culture is obtained, and for example, the description regarding the conversion to Afamin/Wnt3a Conditioned Medium can be cited.

In the present invention, the medium for co-culturing nephron progenitor cells, renal interstitial progenitor cells, and ureteric bud cells may further contain R-spondin 1 (RSPO1). RSPO1 is known to promote Wnt signaling. Therefore, from the above viewpoint, a medium containing a Wnt signal activator, particularly a medium containing a Wnt family protein or a Wnt agonist, preferably further contains RSPO1. RSPO1 is available, for example, from R&D Systems, or may be prepared by oneself. The concentration of RSPO1 in the medium of the present invention is not particularly limited as long as a kidney tissue-like culture is obtained, but may be, for example, 10 ng/mL or more, 20 ng/mL or more, 50 ng/mL or more, 80 ng/mL or more, 100 ng/mL or more, 150 ng/mL or more, 180 ng/mL or more, or 200 ng/mL or more; and may be 1,000 ng/mL or less, 500 ng/mL or less, 400 ng/mL or less, 300 ng/mL or less, 250 ng/mL or less, 220 ng/mL or less, or 200 ng/mL or less; or may be any non-contradictory combination thereof. Specifically, the concentration of RSPO1 in the medium of the present invention may be, for example, 10 ng/mL to 1,000 ng/mL, 20 ng/mL to 500 ng/mL, 50 ng/mL to 400 ng/mL, 100 ng/mL to 300 ng/mL, 150 ng/mL to 250 ng/mL, 180 ng/mL to 220 ng/mL, or 200 ng/mL.

In the present invention, the medium for co-culturing nephron progenitor cells, renal interstitial progenitor cells, and ureteric bud cells may further contain glial cell line-derived neurotrophic factor (GDNF). GDNF is available, for example, from R&D Systems, or may be prepared by oneself. The concentration of GDNF in the medium of the present invention is not particularly limited as long as a kidney tissue-like culture is obtained, but may be, for example, 10 ng/mL or more, 20 ng/mL or more, 50 ng/mL or more, 80 ng/mL or more, 100 ng/mL or more, 150 ng/mL or more, 180 ng/mL or more, or 200 ng/mL or more; and may be 1,000 ng/mL or less, 500 ng/mL or less, 400 ng/mL or less, 300 ng/mL or less, 250 ng/mL or less, 220 ng/mL or less, or 200 ng/mL or less; or may be any non-contradictory combination thereof. Specifically, the concentration of GDNF in the medium of the present invention may be, for example, 10 ng/mL to 1,000 ng/mL, 20 ng/mL to 500 ng/mL, 50 ng/mL to 400 ng/mL, 100 ng/mL to 300 ng/mL, 150 ng/mL to 250 ng/mL, 180 ng/mL to 220 ng/mL, or 200 ng/mL.

In the present invention, the medium for co-culturing nephron progenitor cells, renal interstitial progenitor cells, and ureteric bud cells may further contain FGF9. From the viewpoint of producing a stable kidney tissue-like culture, it is preferred that the medium for co-culturing nephron progenitor cells, renal interstitial progenitor cells, and ureteric bud cells further contains FGF9. FGF9 is available, for example, from Peprotech, or may be prepared by oneself. The concentration of FGF9 in the medium is not particularly limited as long as a kidney tissue-like culture is obtained, but may be, for example, 5 ng/mL or more, 10 ng/mL or more, 25 ng/mL or more, 30 ng/mL or more, 40 ng/mL or more, 50 ng/mL or more, 80 ng/mL or more, or 100 ng/mL or more; and may be 1,000 ng/mL or less, 500 ng/mL or less, 300 ng/mL or less, 200 ng/mL or less, 150 ng/mL or less, 120 ng/mL or less, or 100 ng/mL or less; or may be any non-contradictory combination thereof. Specifically, the concentration of FGF9 in the medium may be, for example, 5 ng/mL to 1,000 ng/mL, 10 ng/mL to 500 ng/mL, 25 ng/mL to 300 ng/mL, 30 ng/mL to 200 ng/mL, 50 ng/mL to 150 ng/mL, 80 ng/mL to 120 ng/mL, or 100 ng/mL.

In the present invention, the medium for co-culturing nephron progenitor cells, renal interstitial progenitor cells, and ureteric bud cells may further contain retinoic acid (RA) and/or a derivative thereof. RA is available, for example, from Sigma, or may be prepared by oneself. The concentration of RA and/or a derivative thereof in the medium is not particularly limited as long as a kidney tissue-like culture is obtained, but may be, for example, 0.005 µM or more, 0.01 µM or more, 0.025 µM or more, 0.03 µM or more, 0.04 µM or more, 0.05 µM or more, 0.08 µM or more, or 0.1 µM or more; and may be 1.0 µM or less, 0.5 µM or less, 0.3 µM or less, 0.2 µM or less, 0.15 µM or less, 0.12 µM or less, or 0.1 µM or less; or may be any non-contradictory combination thereof. Specifically, the concentration of RA and/or a derivative thereof in the medium may be, for example, 0.005 µM to 1.0 µM, 0.01 µM to 0.5 µM, 0.025 µM to 0.3 µM, 0.03 µM to 0.2 µM, 0.05 µM to 0.15 µM, 0.08 µM to 0.12 µM, or 0.1 µM. Examples of the RA derivative include 3-dehydroretinoic acid, 4-[[(5,6,7,8-tetrahydro-5,5,8,8-tetramethyl-2-naphthalenyl)carbonyl]amino]-benzoic acid (AM580) (Tamura K, et al., Cell Differ. Dev. 32: 17-26 (1990)), 4-[(1E)-2-(5,6,7,8-tetrahydro-5,5,8,8-tetramethyl-2-naphthalenyl)-1-propen-1-yl]-benzoic acid (TTNPB) (Strickland S, et al., Cancer Res. 43: 5268-5272 (1983)), and the compounds described in Tanenaga, K. et al., Cancer Res. 40: 914-919 (1980), retinyl palmitate, retinol, retinal, 3-dehydroretinol, 3-dehydroretinal, and the like.

In the present invention, the medium for co-culturing nephron progenitor cells, renal interstitial progenitor cells, and ureteric bud cells may further contain a ROCK (Rho kinase) inhibitor. It is considered that by adding a ROCK inhibitor, cell death can be suppressed and culture, passage, cell proliferation, and/or differentiation can be efficiently performed. From the above viewpoint, the medium of the present invention may contain a ROCK inhibitor, or a ROCK inhibitor may be contained in the medium at the start of cell culture or at the time of passage, and thereafter replaced with a medium not containing a ROCK inhibitor. As the ROCK inhibitor, a known one can be appropriately used, and examples thereof include Y-27632. The concentration of the ROCK inhibitor may be, for example, 1 to 50 µM, and preferably 5 to 20 µM. The ROCK inhibitor is preferably added to the medium for at least 3 days after the start of cell culture or passage.

The number of culture days for co-culturing nephron progenitor cells, renal interstitial progenitor cells, and ureteric bud cells includes, for example, 2 days or more, 3 days or more, 4 days or more, 5 days or more, and 6 days or more. There is no upper limit to the number of culture days, but it may be, for example, 30 days or less. Culture conditions are not particularly limited as long as a kidney tissue-like culture is obtained, but the culture temperature is about 30 to 40°C, preferably about 37°C; the oxygen concentration is a normal oxygen concentration (for example, 15 to 25%, preferably about 20%); and the CO2 concentration is preferably about 2 to 5%.

"Kidney tissue-like culture" refers to a culture having a structure similar to the tissue of an embryonic and/or mature kidney, i.e., a renal tissue structure. Here, a structure similar to a renal tissue structure may mean a structure that is anatomically and/or functionally similar to a renal tissue structure, based on characteristics of each cell type such as marker gene expression and characteristics of the structure formed by those cells, beyond just morphological similarity. That is, "kidney tissue-like culture" may be paraphrased as a kidney organoid. In the embryonic kidney, the metanephros develops from the pronephros through the mesonephros, and the metanephros matures to form an organ corresponding to the kidney. The period when the organ corresponding to the kidney is formed may be referred to as the organogenesis stage. As described above, nephron progenitor cells, renal interstitial progenitor cells, and ureteric bud cells are each one of the progenitor cells for forming the metanephros. Therefore, in the kidney tissue-like culture of the present invention, the embryonic kidney tissue may be metanephros tissue or organogenesis-stage kidney tissue. In other words, the kidney tissue-like culture of the present invention may refer to a culture having a structure similar to the structure of metanephros tissue, organogenesis-stage kidney tissue, and/or mature kidney tissue. The kidney tissue-like culture of the present invention may maintain organogenesis-stage kidney tissue. In one embodiment, the kidney tissue-like culture of the present invention may maintain a nephrogenic zone. Further, the kidney tissue-like culture of the present invention does not exclude having a structure similar to the structure of mature kidney tissue, but does not necessarily have such a structure. That is, the kidney tissue-like culture of the present invention may refer to a culture having a structure similar to the structure of metanephros tissue and/or organogenesis-stage kidney tissue.

Examples of the structure of the renal tissue include, but are not limited to, for example, a mesangial structure, a branched ureteric bud structure, and a cap mesenchyme structure. The kidney tissue-like culture of the present invention preferably has one or more of a mesangial structure, a branched ureteric bud structure, an S-shaped body-like structure, and a cap mesenchyme structure, and more preferably has all of a mesangial structure, a branched ureteric bud structure, and a cap mesenchyme structure. The kidney tissue-like culture of the present invention may further maintain a nephrogenic zone. For example, in the kidney tissue-like culture of the present invention, by maintaining a nephrogenic zone, the formation of further mesangial structures, branched ureteric bud structures, S-shaped body-like structures, and/or cap mesenchyme structures may be promoted.

A mesangial structure refers to a structure formed by podocytes surrounding mesangial cells in a metanephros or a glomerulus of a kidney at the organogenesis stage. A mesangial structure may be characterized by, for example, a structure having mesangial cells inside a glomerular-like structure. Mesangial cells may be characterized by, for example, the expression of a mesangial cell marker gene. Examples of the mesangial cell marker gene include PDGFRB and GATA3. That is, as an example, a mesangial structure may be a structure containing PDGFRB- and/or GATA3-positive cells inside a glomerular-like structure.

A branched ureteric bud structure means a structure derived from a ureteric bud formed by branching and elongation of a ureteric bud. In the present invention, a branched ureteric bud structure preferably has a ureteric bud, and a tip portion of the ureteric bud preferably contains RET-positive cells. More preferably, a branched ureteric bud structure may have two or more ureteric buds, and tip portions of the ureteric buds may contain RET-positive cells. Further, a ureteric bud may be characterized by the expression of the CK8 gene.

An S-shaped body-like structure means an embryonic renal tubule structure formed by epithelialization of nephron progenitor cells. In the present invention, an S-shaped body-like structure preferably includes an S-shaped body-like structure containing a proximal region and a distal region. A proximal region may be characterized by, for example, the expression of HNF4α. A distal region may be characterized by, for example, the expression of BRN1. Note that the distal region may or may not be distinguished from a middle region that is intermediate between the proximal region and the distal region. When the distal region is not distinguished from the middle region, they may be collectively referred to as a mid-distal region. In this case, it may be paraphrased that the S-shaped body-like structure preferably includes an S-shaped body-like structure containing a proximal region and a mid-distal region. A mid-distal region may also be characterized by, for example, the expression of BRN1.

A cap mesenchyme structure refers to a structure of mesenchymal tissue wrapping the tip of a ureteric bud. It is generally known that glomerular podocytes and nephron progenitor cells exist in a cap mesenchyme structure. In the present invention, a cap mesenchyme structure preferably contains nephron progenitor cells and/or cells derived from nephron progenitor cells. Nephron progenitor cells and/or cells derived from nephron progenitor cells may be characterized by, for example, the expression of SIX2 and/or PAX8. More specifically, for example, nephron progenitor cells may be characterized by the expression of SIX2, and cells derived from nephron progenitor cells may be characterized by the expression of PAX8. That is, a cap mesenchyme structure may be a structure that wraps the tip of a ureteric bud and contains SIX2- and/or PAX8-positive cells. Note that a ureteric bud may be characterized by the expression of the CK8 gene. Cells derived from nephron progenitor cells are not particularly limited as long as they are derived from nephron progenitor cells, but may be, for example, cells constituting a metanephric vesicle. Cells constituting a metanephric vesicle may be characterized by, for example, the expression of PAX8. Further, a region characterized by a cap mesenchyme structure containing at least nephron progenitor cells may be regarded as a nephrogenic zone. As described above, nephron progenitor cells may be characterized by the expression of SIX2. In other words, a nephrogenic zone may be observed as a cap mesenchyme structure containing SIX2-expressing cells. That a kidney tissue-like culture maintains a nephrogenic zone may mean that the kidney tissue-like culture contains at least a structure in which the characteristics of the above-mentioned nephrogenic zone are observed. Further, as a non-limiting example, that a kidney tissue-like culture maintains a nephrogenic zone may be that a structure in which the characteristics of the above-mentioned nephrogenic zone are observed is seen during production of the culture, and all or part of such a structure is maintained until after the production of the culture.

### <2> Culture of the present invention

Another aspect of the present invention relates to the kidney tissue-like culture of the present invention. Specifically, in one aspect, the present invention may be: A kidney tissue-like culture, comprising human-derived cells, and having a mesangial structure, a branched ureteric bud structure, an S-shaped body-like structure, and a cap mesenchyme structure.

The method for obtaining the kidney tissue-like culture of the present invention is not particularly limited, but it may be produced by the production method of the present invention.

The kidney tissue-like culture of the present invention may comprise human-derived cells. As an example, when the kidney tissue-like culture of the present invention is obtained by the culture method of the present invention or produced by the production method of the present invention, one or more cells selected from nephron progenitor cells, renal interstitial progenitor cells, and ureteric bud cells may be human-derived cells, and it is preferred that all of the nephron progenitor cells, the renal interstitial progenitor cells, and the ureteric bud cells are human-derived. Further, the kidney tissue-like culture of the present invention preferably consists of human-derived cells.

The kidney tissue-like culture of the present invention may have a mesangial structure, a branched ureteric bud structure, an S-shaped body-like structure, and a cap mesenchyme structure. Details of the mesangial structure, the branched ureteric bud structure, the S-shaped body-like structure, and the cap mesenchyme structure are as described above.

The kidney tissue-like culture of the present invention may be cultured under culture conditions such as a medium in the method of the present invention.

The kidney tissue-like culture of the present invention has a structure similar to the structure of renal tissue, and is expected to gradually undergo organogenesis and develop/grow further as an organ. Therefore, the kidney tissue-like culture of the present invention, which may particularly contain human cells or consist of human cells, can be expected to have various applications such as reconstruction of renal tissue or kidney, regenerative medicine, renal disease models, drug discovery screening, and drug toxicity evaluation systems.

### Examples

The present invention will be specifically described below based on examples, but the present invention is not limited to the following embodiments.

### <Posterior Primitive Streak (PPS) Induction>

Human iPS cells (hiPSC) were first treated with an enzyme using a 1:1 mixture of TrypLE Select Enzyme (Thermo Fisher Scientific) and 0.5 mM EDTA/PBS for 5 minutes, and washed with PBS(-). Thereafter, the cells were detached using a cell scraper, gently pipetted to be dissociated into single cells, and seeded on a 24-well plate (Corning) at a density of 1.3 × 10^4 cells/cm2 together with 0.5 mL of Stem Fit AK02N (Ajinomoto Healthy Supply) supplemented with 10 µM Y-27632 and 2.4 µL/mL iMatrix-511 (Matrixome) 1 day before the start of differentiation. 24 hours later (day 0), culture was started by adding 5 µM CHIR99021 (Wako), 10 nM retinoic acid (RA) (Sigma), 1 ng/mL BMP4 (Peprotech), and 100 ng/mL bFGF (Wako) to a serum-free differentiation medium (hereinafter referred to as the basal medium) consisting of DMEM/F12 Glutamax (Thermo Fisher Scientific), B27 supplement minus vitamin A (Thermo Fisher Scientific), and 0.5× Penicillin/Streptomycin. After another 24 hours (day 1), the cells were cultured in the basal medium containing 5 µM CHIR99021, 100 ng/mL bFGF, and 1 ng/mL BMP7 (R&D Systems). On day 2, the medium was switched to the basal medium containing 5 µM CHIR99021, 1 ng/mL BMP7, and 10 µM A83-01 (Wako). On day 3, the medium was switched to the basal medium containing 5 µM CHIR99021, 30 ng/mL bFGF, 10 ng/mL activin A (R&D Systems), and 30 µM Y-27632 (Wako). On day 4, the cells were washed with PBS(-), treated with Accumax (Innovative Cell Technologies), gently pipetted to be dissociated into single cells, and seeded on a 24-well plate at a density of 1.05 × 10^5 cells/cm2 (2D culture) or seeded on a 96-well low cell-attachment plate (Thermo Fisher Scientific) at a density of 2.0 × 10^4 cells/cm2 (3D culture). The cells were cultured for 48 hours using the basal medium containing 5 µM CHIR99021, 30 ng/mL bFGF, 10 ng/mL activin A, and 30 µM Y-27632 (further containing 1.2 µL/well iMatrix-511 during the 2D culture).

### <Induction of Nephron Progenitor Cells (NPC)>

On day 6 of the PPS induction, the medium was switched to the basal medium containing 0.1 µM RA and 25 ng/mL NOGGIN (Peprotech). On day 8, the medium was switched to the basal medium containing 200 ng/mL FGF9 and 1 µM CHIR99021, and the cells were cultured for 72 hours.

### <Induction of Renal Interstitial Progenitor Cells (IPC)>

On day 4 of the PPS induction, the dissociated cells were seeded in a 96-well U-bottom plate (Sumitomo Bakelite or Thermo Fisher Scientific) at a density of 2.0 × 10^4 cells/well together with the basal medium containing 5 µM CHIR99021, 30 ng/mL bFGF, 10 ng/mL activin A, and 30 µM Y-27632 to form aggregates, and cultured for 48 hours. On day 6, the cells were cultured in an AK02N minus bFGF medium or the basal medium containing 1 µM CHIR99021, 0.1 µM RA, 500 nM Smoothened (SMO) agonist (SAG) (Selleck), and 10 ng/mL IL-1β (Wako). On day 8, the cells were cultured for 72 hours in the AK02N minus bFGF medium or the basal medium containing 1 µM CHIR99021, 0.1 µM RA, 500 nM SAG, and 10 µM SB431542.

### <Induction of Nephric Duct (ND) Cells (Ureteric Bud Cells)>

First, anterior intermediate mesoderm was induced from hiPSCs by the following method. hiPSCs were treated by an enzymatic method using a 1:1 mixture of TrypLE Select Enzyme and 0.5 mM EDTA/PBS for 5 minutes, and washed with PBS(-). Next, the cells were detached using a cell scraper and separated into single cells by gentle pipetting. One day before starting differentiation, seeding was performed on a 10-cm dish (Falcon) at a density of 4.0 × 10^5 cells/dish using Stem Fit AK02N (supplemented with 10 µM Y-27632 and 20 µL/dish iMatrix-511). After 24 hours (day 0), the cells were cultured in a serum-free differentiation medium in which 50 ng/mL activin A, 5 µM CHIR99021, 25 ng/mL BMP4, and 25 ng/mL bFGF were added to Essential 6 medium (E6; Thermo Fisher Scientific). After about 22 hours (day 1), the cells were cultured for 2 days by adding 100 nM LDN193189, 1 µM A83-01, 0.1 µM TTNPB (Santa Cruz Biotechnology), and 200 ng/mL FGF8 (Peprotech) to the E6 medium. Thereafter, the cells were re-seeded on a 10-cm dish at a density of 8.7 × 10^6 cells/dish under conditions containing the same 4 factors and 10 µM Y-27632 in the E6 medium, and further cultured for 24 hours.

Next, ND cells were induced from the anterior intermediate mesoderm by the following method. The anterior intermediate mesoderm was treated for 2 days with an E6 medium containing 1 µM CHIR99021, 100 nM LDN193189, 200 ng/mL FGF8, 100 ng/mL GDNF, and 0.1 µM TTNPB, then treated with Accutase (Nacalai tesque) at 37°C for 3 minutes, separated into single cells by pipetting, and seeded in a low-attachment 96-well plate (Sumitomo Bakelite) at a density of 1.0 × 10^4 cells/well. Then, the cells were cultured for 2 days under conditions containing 10 µM Y-27632 in addition to the same medium and factors to induce ND cells.

### <Reconstruction of Kidney Structure>

The NPC, IPC, and ND cells induced as described above were left standing with Accumax at 37°C for 10 minutes, and gently pipetted to be dissociated into single cells. The dissociated single cells were mixed and resuspended in a basal medium containing 1 µM CHIR99021, 0.1 µM RA, 100 ng/mL FGF9, and 10 µM Y-27632 (CRFY), a medium in which 200 ng/mL GDNF was further added to CRFY (CRFY+G), or a basal medium (RFY+G+WR) containing 0.1 µM RA, 100 ng/mL FGF9, and 10 µM Y-27632, 200 ng/mL GDNF, and 10% Afamin/Wnt3a Conditioned Medium (MBL; J-ORMW301R) and 200 ng/mL R-spondin1 protein (RSPO1) (R&D), and seeded in a 96-well low cell-binding U-bottom plate at a density of 1.7 × 10^4 cells/well for NPCs, 1.7 × 10^4 cells/well for IPCs, and 1.7 × 10^4 cells/well for ND cells to form aggregates. After 48 hours, the mixed aggregates were transferred to a 24-well plate and left standing at 37°C for 30 minutes together with the same medium as above supplemented with 20 µL of 50% Matrigel. After some degree of coagulation, 180 µL of the same medium as above was added. Then, half of the medium was exchanged every 2 days.

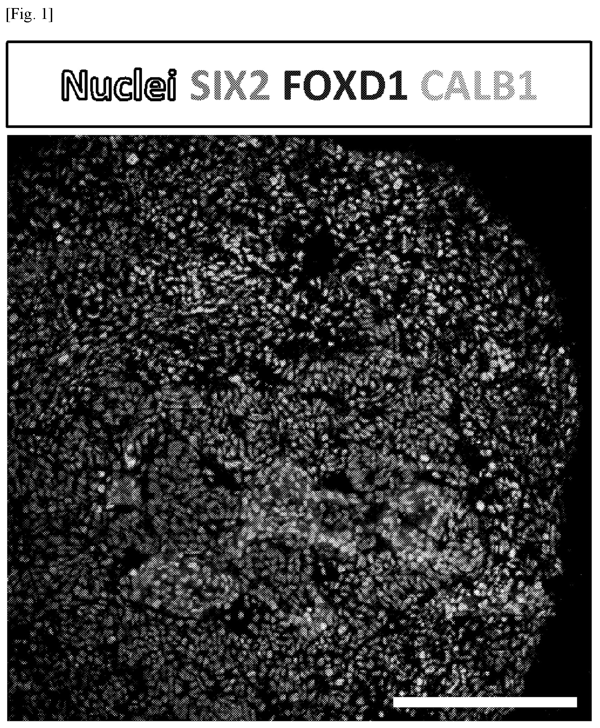

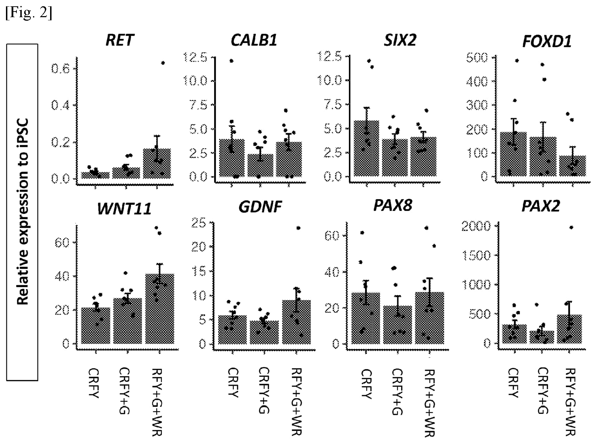

As a result of analyzing a tissue-like culture on day 6 after kidney structure reconstruction was performed under CRFY conditions by immunostaining, a CALB1(+) epithelial structure was reconstructed, and SIX2(+) NPCs and FOXD1(+) IPCs surrounded a ureteric bud (UB)-like epithelial structure (FIG. 1), but RET expression was not observed in the structure similar to a UB tip. The medium used for cell proliferation at the tip of the ureteric bud in a previous report (Mae, S.I., et al. Cell Rep. 32. 2020) contained GDNF. Therefore, kidney structure reconstruction was performed under CRFY+G conditions in which GDNF was added to CRFY. In the tissue-like culture on day 6 under these conditions, as a result of analysis by quantitative RT-PCR (qT-PCR), RET expression was slightly increased, and other progenitor cell markers such as SIX2 and FOXD1 were not affected (FIG. 2).

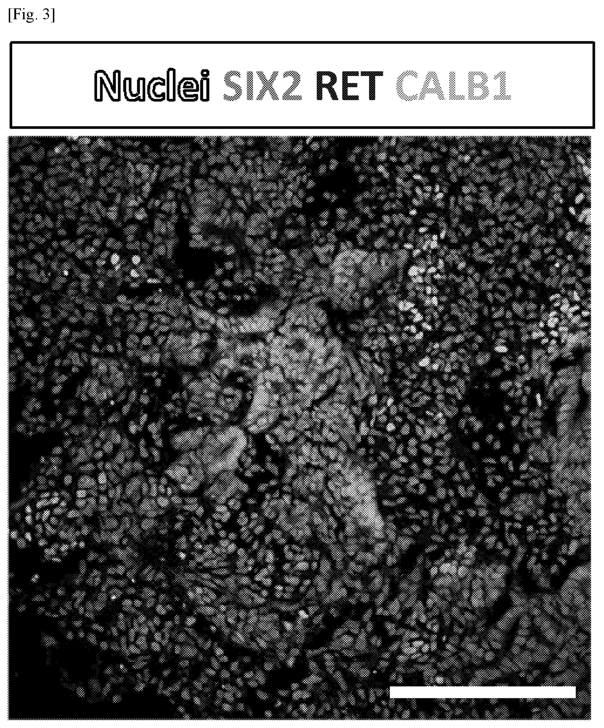

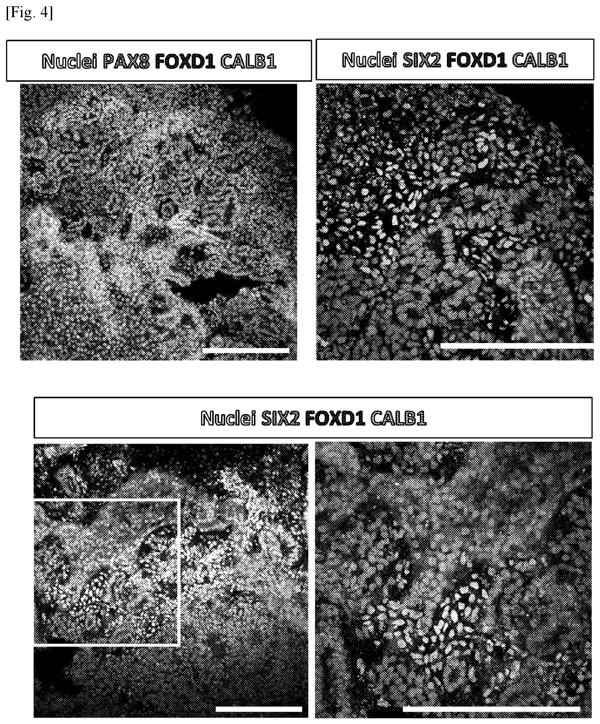

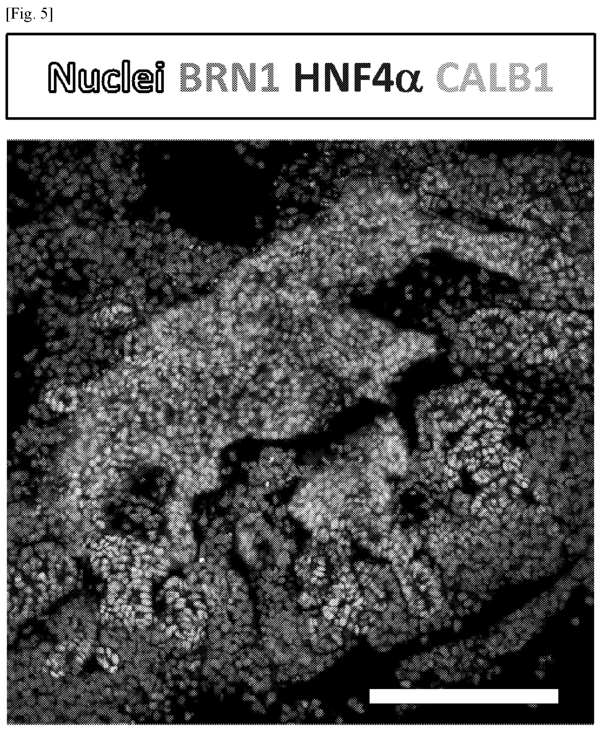

On the other hand, in a tissue-like culture on day 6 after kidney structure reconstruction was performed under RFY+G+WR conditions in which GDNF was added to CRFY and Wnt3a and RSPO1 were used instead of CHIR99021, according to analysis by qRT-PCR, RET expression was further improved compared to that under CRFY+G conditions (FIG. 2). Further, as a result of analyzing the culture by immunostaining, the sprouted UB tip was RET-positive (FIG. 3), and nephrogenic zone-like structures, for example, a SIX2(+) or PAX8(+) cap mesenchyme structure (FIG. 4) and an S-shaped body-like structure consisting of an HNF4α(+) proximal region and a BRN1(+) mid-distal region (FIG. 5), were formed.

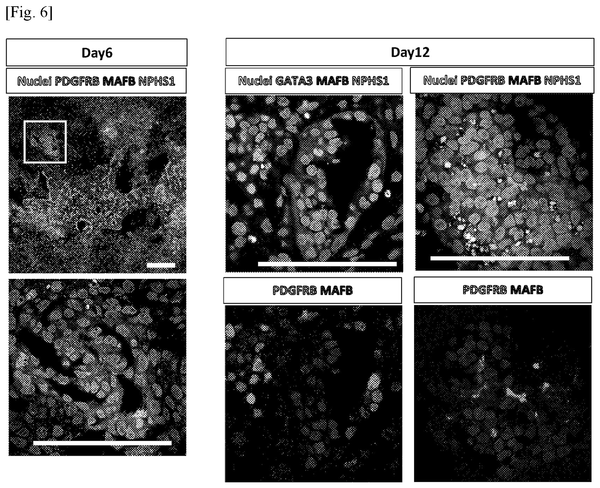

Mesangial cells are derived from Foxd1(+) IPCs and migrate in the glomerular capillary loops during development. In the glomeruli of embryonic kidneys at the capillary loop stage, podocytes surround Pdgfrb(+) mesangial cells to form a mesangial structure. Consistent with this, in tissues corresponding to the capillary loop stage on days 6 and 12 and in tissues corresponding to the maturation stage on day 12 after kidney structure reconstruction was performed under RFY+G+WR conditions, a glomerular-like structure having PDGFRB(+) or GATA3(+) mesangial-like cells, i.e., a mesangial structure, was observed (FIG. 6). Note that when IPCs constantly expressing green fluorescent protein (EGFP) and non-fluorescent NPCs were cultured under RFY+G+WR conditions, an EGFP(+)PDGFRB(+) mesangial-like structure was observed, and it was confirmed that mesangial-like cells were derived from IPCs.

From the above, by co-culturing NPC cells, IPC cells, and ND cells under RFY+G+WR conditions, an appropriate UB structure, nephrogenic zone, and mesangial structure could be formed. That is, by co-culturing NPC cells, IPC cells, and ND cells (ureteric bud cells) under culture conditions containing Wnt3a, which is a Wnt signal activator, a kidney tissue-like culture having the structure of a kidney at the organogenesis stage was produced.

### <Evaluation of Nephron Progenitor Cells (NPC)>

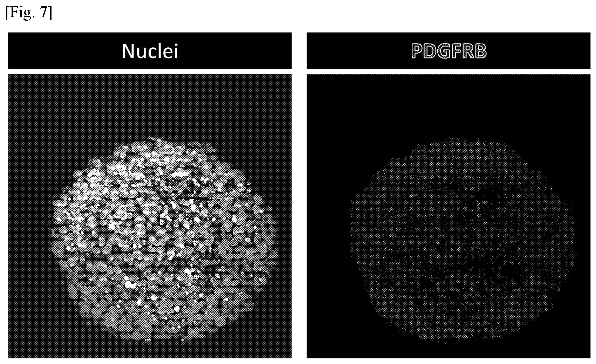

The NPCs induced by the above <Induction of Nephron Progenitor Cells (NPC)> were left standing with Accumax at 37°C for 10 minutes, and gently pipetted to be dissociated into single cells. The dissociated single cells were mixed, resuspended in a basal medium (CRFY) containing 1 µM CHIR99021, 200 ng/mL FGF9, and 10 µM Y-27632, and seeded in a 96-well low cell-binding U-bottom plate at a density of 4 × 10^4 cells/well to form aggregates. After 48 hours, the cell masses were collected, frozen sections were prepared, and fluorescent immunostaining of a marker gene for interstitial progenitor cells was performed. As a result, it was shown that interstitial progenitor cells indicated by PDGFRB(+) were partially included (FIG. 7).

### <Induction of Ureteric Bud Organoids>

Next, ureteric bud organoids were induced from nephric duct (ND) cells (ureteric bud cells) by the following method. After washing with PBS, nephric duct (ND) cells (ureteric bud cells) were treated with an E6 medium containing 1 µM CHIR99021, 100 nM LDN193189, 200 ng/mL FGF8, 100 ng/mL GDNF, 0.1 µM TTNPB, 200 ng/mL FGF1, 50 ng/mL EGF, and 2% Matrigel, and cultured for 7 days to induce ureteric bud organoids.

### <Reconstruction of Kidney Structure by Ureteric Bud Organoids and NPC>

The NPCs induced as described above were left standing with Accumax at 37°C for 10 minutes, and gently pipetted to be dissociated into single cells. The dissociated single-cell NPCs were resuspended in a basal medium (RFY+G+WR) containing 0.1 µM RA, 100 ng/mL FGF9, and 10 µM Y-27632, 200 ng/mL GDNF, and 10% Afamin/Wnt3a Conditioned Medium and 200 ng/mL R-spondin1 protein (RSPO1). A suspension of NPCs at a density of 5 × 10^4 cells/well was seeded from above so as to sprinkle over the ureteric bud organoids induced as described above, thereby forming aggregates. After 48 hours, the mixed aggregates were transferred to a 24-well plate and left standing at 37°C for 30 minutes together with the same medium as above supplemented with 20 µL of 50% Matrigel. After some degree of coagulation, 180 µL of the same medium as above was added. Then, half of the medium was exchanged for the basal medium every 2 days.

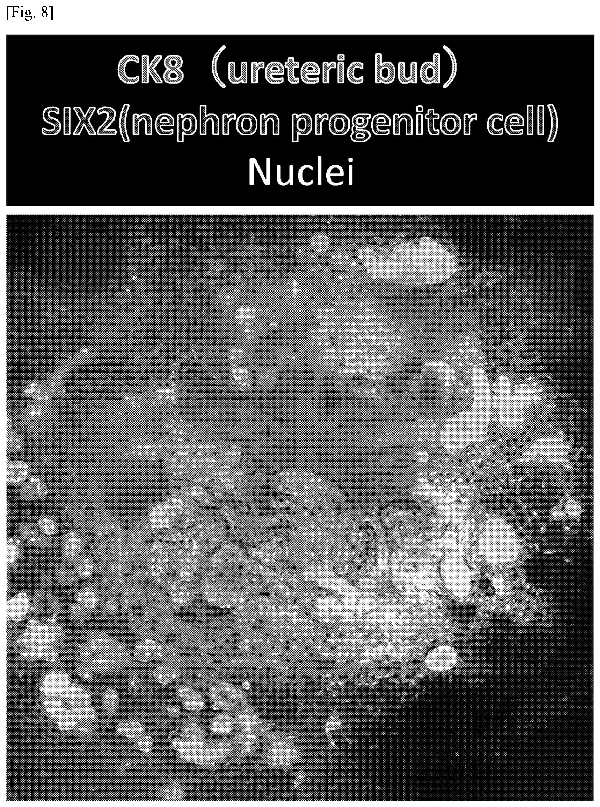

As a result of analyzing a tissue-like culture on day 13 after kidney structure reconstruction was performed under RFY+G+WR conditions by immunostaining, a kidney tissue having a CK8(+) branched ureteric bud structure was produced while maintaining a cap mesenchyme structure of SIX2(+) nephron progenitor cells and maintaining a nephrogenic zone (FIG. 8).

From the above, by co-culturing NPC cells (partially including IPC cells) with ureteric bud organoids under RFY+G+WR conditions, an appropriate UB structure and nephrogenic zone could be formed. That is, by co-culturing NPC cells, IPC cells, and ureteric bud organoids (ureteric bud cells) under culture conditions containing Wnt3a, which is a Wnt signal activator, a kidney tissue-like culture having the structure of a kidney at the organogenesis stage was produced.

## Claims

1. A method for producing a kidney tissue-like culture, comprising a step of co-culturing nephron progenitor cells, renal interstitial progenitor cells, and ureteric bud cells in a medium containing a Wnt signal activator.

2. The method according to claim 1, wherein the co-culture is performed by suspension culture.

3. The method according to claim 2, wherein the suspension culture is performed in a state where an aggregate of nephron progenitor cells, renal interstitial progenitor cells, and ureteric bud cells is coated with an extracellular matrix.

4. The method according to claim 1, wherein the Wnt signal activator is a Wnt3a protein.

5. The method according to claim 1, wherein the medium further contains fibroblast growth factor 9 (FGF9).

6. The method according to any one of claims 1 to 5, wherein the medium further contains retinoic acid (RA) and/or a derivative thereof, and glial cell line-derived neurotrophic factor (GDNF).

7. The method according to claim 6, wherein the medium further contains alpha-albumin and R-Spondin 1 (RSPO1).

8. The method according to any one of claims 1 to 5, wherein the kidney tissue-like culture has one or more of a mesangial structure, a branched ureteric bud structure, an S-shaped body-like structure, and a cap mesenchyme structure.

9. The method according to any one of claims 1 to 5, wherein the nephron progenitor cells, the renal interstitial progenitor cells, and the ureteric bud cells are derived from a human.

10. The method according to any one of claims 1 to 5, wherein one or more of the nephron progenitor cells, the renal interstitial progenitor cells, and the ureteric bud cells are cells induced from pluripotent stem cells.

11. A kidney tissue-like culture,
comprising human-derived cells and
having a mesangial structure, a branched ureteric bud structure, an S-shaped body-like structure, and a cap mesenchyme structure.

12. The kidney tissue-like culture according to claim 11, wherein:
the mesangial structure is a structure containing PDGFRB- and/or GATA3-positive cells inside a glomerular-like structure;
the branched ureteric bud structure is a structure having two or more ureteric buds, wherein a tip portion of the ureteric bud contains RET-positive cells;
the S-shaped body-like structure is a structure including an S-shaped body-like structure containing a proximal region and a distal region; and
the cap mesenchyme structure is a structure that wraps the tip of the ureteric bud and contains SIX2- and/or PAX8-positive cells.
